# NEUE EUROPÄISCHE PATENTSCHRIFT

(11) **EP 1 149 840 B2**
(45) Veröffentlichungstag und Bekanntmachung des Hinweises auf die Entscheidung über den Einspruch: **15.05.2013**
(45) Hinweis auf die Patenterteilung: 07.01.2009
(21) Anmeldenummer: 01250277.9
(22) Anmeldetag: 11.08.1997
(51) Int. Cl.: C07J 53/00

(54) **Verfahren zur Herstellung von Drospirenon**
Process for preparation of drospirenon
Procédé de préparation de drospirenon

(30) Priorität: 12.08.1996 DE 19633685
(43) Veröffentlichungstag der Anmeldung: 31.10.2001
(62) Teilanmeldung aus: 97937562.3
(73) Patentinhaber: Bayer Pharma Aktiengesellschaft, 13353 Berlin (DE)
(72) Erfinder: Mohr, Jörg-Thorsten, 59423 Unna (DE); Nickisch, Klaus, 12307 Berlin (DE)
(74) Vertreter: König, Gregor Sebastian

(56) Entgegenhaltungen:
- EP-A- 0 051 143
- EP-A- 0 075 189
- DE-A- 2 652 761
- DE-A- 3 626 838
- US-A- 4 416 985
- NICKISCH K ET AL: "ACID CATALYZED REARRANGEMENTS OF 15-BETA 16-BETA METHYLENE-17-ALPHA-PREGNENE-21 17-CARBOLACTONE DERIVATIVES" TETRAHEDRON LETTERS, Bd. 27, Nr. 45, 1986, Seiten 5463-5466, XP002298941 ISSN: 0040-4039
- BITTLER D ET AL: "SYNTHESIS OF SPIRORENONE - A NOVEL HIGHLY ACTIVE ALDOSTERONE ANTAGONIST" ANGEWANDTE CHEMIE. INTERNATIONAL EDITION, VERLAG CHEMIE. WEINHEIM, DE, Bd. 21, Nr. 9, 1982, Seiten 696-697, XP002047531 ISSN: 0570-0833

## Beschreibung

Die Erfindung betrifft ein Verfahren zur Herstellung von Drospirenon (6β,7β; 15β,16β-Dimethylene-3-oxo-17α-pregn-4-ene-21,17-carbolactone, **DRSP**.

Drospirenon (6β,7β; 15β,16β-dimethylene-3-oxo-17α-pregn-4-ene-21,17-carbolactone, **DRSP,** INN) ist als steroidaler Wirkstoff seit längerem bekannt (DE 26 52 761 C2 und DE 30 22 337 A1) und die Herstellung der letzten 4 Schritte erfolgt im Eintopfverfahren; bei dem nach der Hydrierung von Dimethylenpropinol keine der durchlaufenen Zwischenstufen Dimethylenpropanol und 5-β-OH-DRSP isoliert werden (siehe nachfolgendes Schema).

Eine analoge Synthese, jedoch unter Anwendung einer Pyridiniumdichromat-Oxidation ist aus dem Stand der Technik bekannt [Angew. Chemie, 21, 9, (1982), Seiten 696 - 697]. Ähnliche Synthesen zur Herstellung von steroidalen 17, 21-Carbolactonen werden auch innerhalb von EP-A-0 075 189 und EP-A-0 051 143 beschrieben, jedoch unter Beteiligung von mikrobiologischen Reaktionen. Oxidationen unter Beteiligung von Rutheniumverbindungen werden aber nicht darin offenbart.

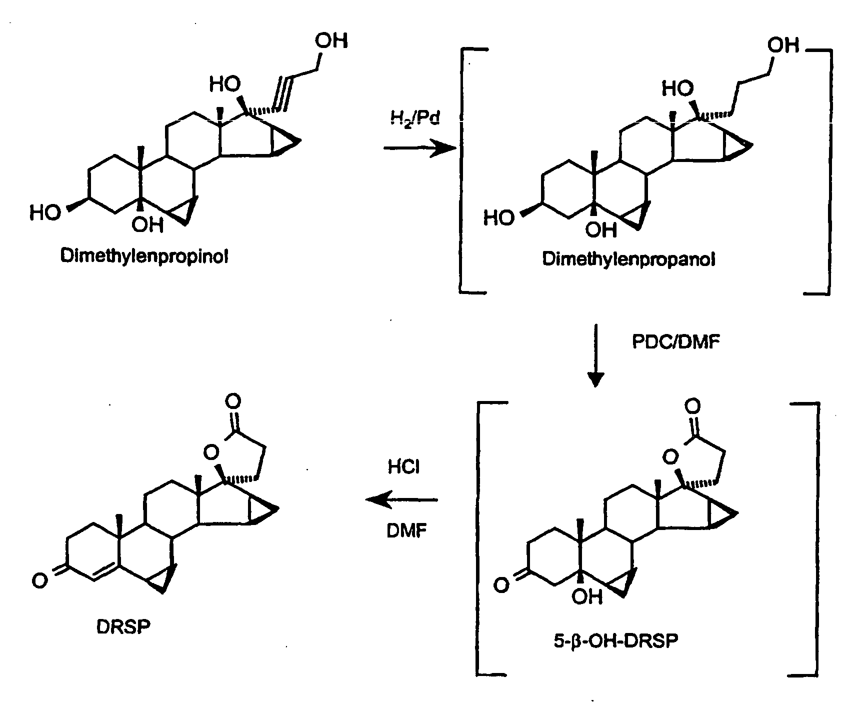

Das Dimethylenpropinol wird in THF mit Wasserstoff an Palladium-Kohle zum Dimethylenpropanol hydriert. Die so erhaltene Hydrierlösung, die das Propanol als Hauptprodukt und schwankende Anteile an Lactol enthält, wird ohne Isolierung und Zwischenaufarbeitung zum Drospirenon (DRSP) umgesetzt.

Hierzu wird zuerst ein Lösungsmittelwechsel von THF zu DMF vollzogen und anschließend das Propanol bei 40° C mit einem Überschuß von 3,7 Equivalenten Pyridiniumdichromat (PDC) zu einem Gemisch von DRSP und 5-β-OH-DRSP oxidiert. Die 5-β-OH-Funktion im Oxidationsprodukt ist labil gegenüber Säuren, Lewissäuren und basischen Bedingungen bei erhöhten Temperaturen, da in allen Fällen mit der Ausbildung des Δ-4,5-ungesättigten Ketons im Drospirenon ein thermodynamisch stabileres Produkt erhalten wird. Die Eliminierung der β-OH-Funktion im 5-β-OH-DRSP verläuft zum thermodynamisch stabileren Drospirenon und konnte nicht unterdrückt werden konnte.

Die Mischung enthält in der Regel wechselnde Anteile der beiden Komponenten, wobei das 5-β-OH-DRSP im allgemeinen als Hauptkomponente im Verhältnis von 2-3:1 vorliegt. In der letzten Stufe der Eintopfsequenz wird das Zweikomponenten-Gemisch durch Zugabe von halbkonzentrierter Salzsäure in das DRSP, roh überführt.

In der Nachstehenden Tabelle sind die letzten vier Betriebsansätze zusammengefaßt.

| Ansatz | Ausbeute, roh (%) | Reinheit (100%-Methode) |
|---|---|---|
| 1 | 57,2 | 98,9 |
| 2 | 63,7 | 99,09 |
| 3 | 46,5 | 99,18 |
| 4 | 58,3 | 98,81 |
| **Gesamt** | **mittlere Ausbeute: 56,4** | **mittlere Reinheit: 98,9** |

Im Mittel aller Betriebsansätze wird ausgehend vom Dimethylenpropinol eine theoretische Ausbeute von 56% DRSP, roh in einer HPLC-Reinheit von 98,9% erzielt.

Aufgabe der Erfindung ist die Bereitstellung eines neuen Herstellungsverfahrens für Drospirenon.

Gelöst wird diese Aufgabe gemäß der Lehre des Ansprüchs. Die in der Stammanmeldung beschriebene Erfindung beinhaltet ein Verfahren zur Herstellung von Drospirenon (6β,7β; 15β,16β-dimethylene-3-oxo-17α-pregn-4-ene-21,17-carbolactone, **DRSP)** 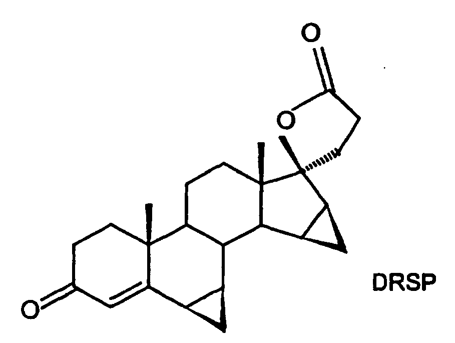 durch katalytische Hydrierung von 17α-(3-hydroxy-1-propynyl)-6β,7β;15β,16β-dimethylene-5β-androstane-3β,5,17β-triol 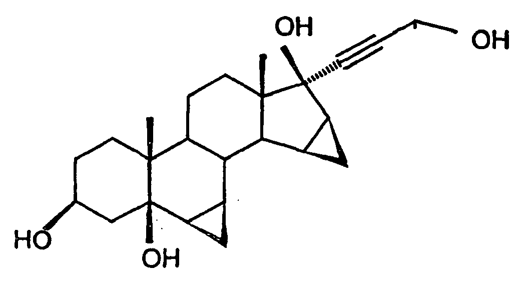 in das 7α-(3-hydroxy-1-propyl)-6β,7β; 15β,16β-dimethylene-5β-androstane-3β,5,17β-triol,

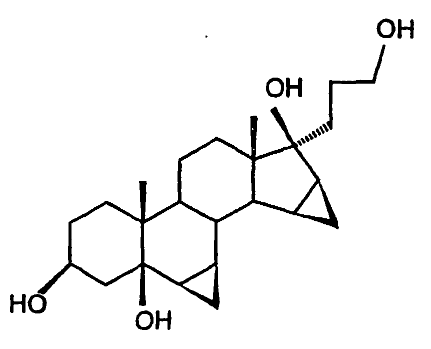

Oxidation in Gegenwart eines Rutheniumsalzes in das 6β,7β;15β,16β-dimethylene-5 β-hydroxy-3-oxo-17α-androstane-21,17-carbolactone 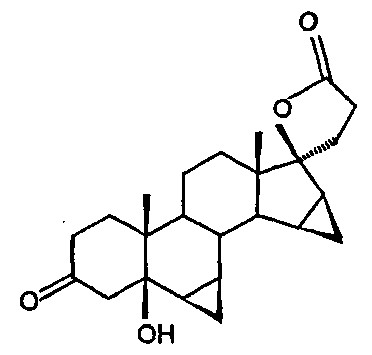 und anschließender Wasserabspaltung.

Die Erfindung beinhaltet die Wassereliminierung von 5β-OH-DRSP zum Drospirenon gemäß Anspruch.

Analog zum bekannten Verfahren aus dem Stand der Technik wird in dem in der Stammanmeldung beschriebenen Verfahren Dimethylenpropinol in THF mit Wasserstoff an Palladium-Kohle hydriert. Die Hydrierlösung wird anschließend einem Lösungsmittelwechsel von THF auf Acetonitril unterworfen. Die Acetonitril-Lösung wird mit einer katalytischen Menge Rutheniumtrichlorid (1mol%) und 3 Equivalenten Natriumbromat bei 40°-60° C gezielt zum 5-β-OH-DRSP oxidiert.
Trotz der großen Labilität des 5-β-OH-DRSP gegenüber Säuren, Lewissäuren wie beispielsweise der Chromverbindungen im alten Betriebsverfahren, starken Basen oder hohen Temperaturen, die in allen Fällen auf die hohe Triebkraft zur Bildung des thermodynamisch stabileren Δ-4,5-ungesättigten Ketons zurückzuführen ist, gelingt unter den in der Stammanmeldung gewählten Reaktionsbedingungen die selektive Synthese des 5-β-OH-DRSP, ohne das eine Drospirenonbildung zu beobachten ist. Das 5-β-OH-DRSP kann durch eine (betrieblich) einfach durchzuführende Wasserfällung aus der Reaktionslösung isoliert werden.

Die Ausbeuten liegen im Bereich von 68% bis 75% über die beiden Stufen Hydrieren und anschließende Oxidation.

Aus eigenen Versuchen ist bekannt, daß das Drospirenon bei Säureeinwirkung nach zwei Reaktionswegen zersetzt werden kann. Einmal wird das Drospirenon unter sauren Bedingungen leicht in das epimere Isolacton überführt.

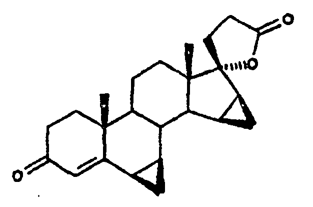

Das zweite Nebenprodukt entsteht durch einen HCl-Angriff auf die 6,7-Methylengruppe, der zu dem Ringöffnungsprodukt führt.

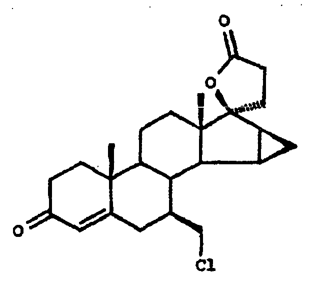

Beide Nebenprodukte, das Isolacton und das durch Säureeinwirkung gebildete Ringöffnungsprodukt, werden unter den Reaktionsbedingungen des neuen Verfahrens soweit zurückgedrängt, daß sie nur noch in einer Größenordnung von <0,2% zu beobachten sind.

Bei der Eliminierung wird eine Ausbeute von 96% d.Th. erzielt.

### Beispiele

### 6β,7β;15β,16β-dimethylene-5β-hydroxy-3-oxo-17α-androstane-21,17-carbolactone

50 g 17α-(3-Hydroxy-1-propynyl)-6β,7β; 15β,16β-dimethylene-5β-androstane-3β ,5,17β-triol wird in 1000 ml THF in der Gegenwart von 10 g Palladium auf Kohle (10%) und 3 ml Pyridin hydriert bis 2 Equivalente Wasserstoff aufgenommen werden. Anschließend wird der Katalysator abfiltriert und die Lösung bis zur Trockne eingeengt, wobei 52,7 g 7α(3-hydroxy-1-propyl)-6β,7β; 15β,16β-dimethylene-5β-androstane-3β,5,17β-triol erhalten werden, die ohne Reinigung weiter umgesetzt werden.

50, 2 g 7α-(3-hydroxy-1-propyl)-6β,7β; 15β,16β-dimethylene-5β-androstane-3β,5,17β -triol werden in 250 ml Acetonitril suspendiert und auf 45° C erwärmt. Hierzu werden 0,52g Rutheniumtrichlorid gelöst in 10 ml Wasser und 62,46 g Natriumbromat gelöst in 250 ml Wasser getropft. Es wird 2 Stunden bei 50° C nachgerührt und die Lösung anschließend durch Zugabe von 1000 ml Wasser gequencht. Es werden 200 ml Ethylacetat zugesetzt, die Phasen getrennt und anschließend die wässrige Phase mit 600 ml Ethylacetat extrahiert. Die vereinigten organischen Phasen werden über Natriumsulfat getrocknet und anschließend bis zur Trockne eingeengt. Hierbei werden 43,44 g 6β,7β;15β,16β-Dimethylene-5β-hydroxy-3-oxo-17α-androstane-21,17-carbolactone als Rohprodukt erhalten. Durch Umkristallisieren aus Aceton-lsoether erhält man 35,7g 6β,7β;15β,16β-Dimethylene-5β-hydroxy-3-oxo-17α-androstane-21,17-carbolactone mit einem Schmelzpunkt von 216°-218°C. Der Drehwert liegt bei -65,6° (Natriumlinie, c=1,02 in CHCI3).

### 6β,7β; 15β,16β-Dimethylene-3-oxo-17α-pregn-4-ene-21,17-carbolactone

28 g 6β,7β;15β,16β-dimethylene-5β-hydroxy-3-oxo-17α-androstane-21,17-carbolactone werden in 280 ml THF suspendiert und anschließend mit 10 mol%, 1,5g p-Toluolsulfonsäure versetzt. Nach 30 Minuten werden 125 ml ges. NaCI-Lösung und 8,2 ml 1n NaOH-Lösung zugegeben. Nach Phasentrennung wird die organische Phase über Natriumsulfat getrocknet und bis zur Trockne eingeengt, wobei 25,67g 6 β,7β; 15β,16β-dimethylene-3-oxo-17α-pregn-4-ene-21,17-carbolactone als Rohprodukt erhalten, dessen Reinheit nach HPLC-Bestimmung bei 93% liegt.

Der Schmelzpunkt der chromatographierten Substanz liegt bei 197,5° -200° C.

## Patentansprüche

1. Verfahren zur Herstellung von Drospirenon (6β,7β;15β,16β-dimethylen-3-oxo-17a-pregn-4-ene-21,17-carbolacton, **DRSP**) durch Wasserabspaltung aus 6β,7β;15β,16β-dimethylen-5 β-hydroxy-3-oxo-17α-androstan-21,17-carbolacton durch Zugabe von p-Toluolsulfonsäure zu 6β,7β;15β,16β-dimethylen-5 β-hydroxy-3-oxo-17a-androstan-21,17-carbolacton.

## Claims

1. Process for preparing drospirenone (6β,7β;15β,16β-dimethylene-3-oxo-17a-pregn-4-ene-21,17-carbolactone, **DRSP**) by water elimination from 6β,7β;15β,16β-dimethylene-5β-hydroxy-3-oxo-17α-androstane-21,17-carbolactone through addition of p-toulenesulphonic acid to 6β,7β;15β,16β-dimethylene-5β-hydroxy-3-oxo-17α-androstane-21,17-carbolactone.

## Revendications

1. Procédé de préparation de la drospirénone (6β,7β;15β,16β-diméthylène-3-oxo-17a-prégn-4-ène-21,17-carbolactone, **DRSP**) par l'élimination d'eau à partir de la 6β,7β;15β,16β-diméthylène-5β-hydroxy-3-oxo-17a-androstan-21,17-carbolactone par l'addition d'acide p-toluènesulfonique à la 6β,7β;15β,16β-diméthylène-5β-hydroxy-3-oxo-17α-androstan-21,17-carbolactone.
